(19) Europäisches Patentamt

European Patent Office

Office européen des brevets

(11) **EP 1 509 285 B1**

(12) **EUROPEAN PATENT SPECIFICATION**

(45) Date of publication and mention
of the grant of the patent:
**20.12.2006 Bulletin 2006/51**

(51) Int Cl.:
*A61P 1/16* (2006.01)      *A61K 31/12* (2006.01)
*A61K 31/19* (2006.01)      *A61K 36/00* (2006.01)

(21) Application number: **02722566.3**

(22) Date of filing: **25.03.2002**

(86) International application number:
**PCT/IB2002/001212**

(87) International publication number:
**WO 2003/080081 (02.10.2003 Gazette 2003/40)**

(54) **SYNERGISTIC COMPOSITION OF TRANS-TETRACOS-15-ENOIC ACID AND APOCYNIN AND USE THEREOF**

SYNERGISTISCHE ZUSAMMENSETZUNG AUS TRANS-TETRACOS-15-ENSÄURE UND APOCYNIN, UND VERWENDUNG DAVON

COMPOSITION SYNERGISTIQUE D'ACIDE 15 TRANSTETRACOSENOIQUE ET UTILISATION ASSOCIEE

(84) Designated Contracting States:
**AT DE FR GB IT**

(43) Date of publication of application:
**02.03.2005 Bulletin 2005/09**

(73) Proprietor: **COUNCIL OF SCIENTIFIC AND INDUSTRIAL RESEARCH**
**New Delhi 110 001 (IN)**

(72) Inventors:
• **HANDA, Sukhdev Swami**
  **Jammu 180 001 (IN)**
• **SURI, Om, Parkash**
  **Jammu 180 001 (IN)**
• **GUPTA, V.N.**
  **Jammu 180 001 (IN)**
• **SURI, Krishan, Avtar**
  **Jammu 180 001 (IN)**
• **SATTI, Naresh, Kumar**
  **Jammu 180 001 (IN)**
• **BHARDWAJ, Vikram**
  **Jammu 180 001 (IN)**
• **SINGH, Bupinder**
  **Jammu 180 001 (IN)**
• **CHANDAN, Bal, Krishan**
  **Jammu 180 001 (IN)**

(74) Representative: **Becker Kurig Straus**
**Patentanwälte**
**Bavariastrasse 7**
**80336 München (DE)**

(56) References cited:
**WO-A-02/04003**          **WO-A-99/12539**
**GB-A- 2 368 012**

• **ANAND ET AL: "Protective effect of alcoholic extract of Indigofera tinctoria Linn. in experimental lliver injury" INDIAN JOURNAL OF EXPERIMENTAL BIOLOGY, vol. 17, no. 7, July 1979 (1979-07), pages 685-687, XP008016611 cited in the application**
• **RRL JAMMU RESEARCH CENTRE, [Online] XP002239717 Retrieved from the Internet: < URL:http://www.rrljammu.org/downloads/patents_filed2001.pdfhttp://www.rrljammu.org/downloads/patents_filed2001.pdfhttp://www.rrljammu.org/downloads/patents_filed2001.pdf> [retrieved on 2003-04-28]**
• **LEE, EUN JU ET AL: "Hepatoprotective phenylpropanoids from Scrophularia buergeriana roots against CCl4-induced toxicity: action mechanism and structure-activity relationship" PLANTA MEDICA (2002), 68(5), 407-411 , XP008016716**
• **LOMNITSKI, LIAT ET AL: "Effects of apocynin and natural antioxidant from spinach on inducible nitric oxide synthase and cyclooxygenase-2 induction in lipopolysaccharide-induced hepatic injury in rat" PHARMACOLOGY & TOXICOLOGY (COPENHAGEN) (2000), 87(1), 18-25 , XP008016718**

- O'NEIL ET AL, EDS.: "The Merck Index" 2001 , MERCK RESEARCH LABORATORIES OF MERCK & CO., INC. , WHITEHOUSE STATION, NJ XP002239718 page 125, right-hand column, paragraphs 746-748

- O'NEIL ET AL, EDS.: "The Merck Index" 2001 , MERCK RESEARCH LABORATORIES OF MERCK & CO., INC. , WHITEHOUSE STATION, NJ XP002239718 page 125, right-hand column, paragraphs 746-748

**Description**

**TECHNICAL FIELD**

The present invention relates to a synergistic hepatoprotective composition comprising trans-tetracos-15-enoic acid (TCA) and Apocynin (APO). The present invention also relates to use in the treatment of hepatotoxicity in mammals.

**BACKGROUND ART**

Apocynin, a cardiotonic constituent of the rhizomes of Apocyanum cannabium [Finnemore Horace, J. Chem. Soc. 93 (1908) 1513-1520] and A. androsaemifolium [Naves Yves-Rene, Helv Chim Acta 32 (1949) 1351] and essential oils of the rhizomes of Iris species have been reported to be responsible for choleretic activity of Picrorhiza kurroa [Basu, K., Dasgupta B., Bhattacharya, S.K. and Debnath, P.K. Curr Sci, 40 (1971) 603]. The constituent has been synthesized and pharmacologically evaluated [Suri, O.P., Bindra, R.S., Satti, N.K. and Khajuria, R.K. Indian Journal of Chemistry, 26B (1987), p 587-88]. Apocynin has also been evaluated for antioxidant and free radical scavenging activities.

Roots of Picrorhiza kurroa are used therapeutically in traditional medicine of almost all Asian countries to treat a manifold of conditions of illness including liver, lung & spleen ailments [Rajaram, D. (1976) Bomb. Hosp. Journal, 18, 66-69; Pandey, G.S. (1979) Indian Materia Medica, Chaukhamba Sanskrit Sansthan, Varanasi pp 70-71; Langar, J.G., Gupta, O.P. and Atal, C.K. (1981), Ind. J. Pharm. 13, 98-99; Handa, S.S., Sharma, A. and

Chakraborty, K.K. (1986), Fitotherapia, 58, 307-351; Ansari, R.R., Kapoor, N.K., Kulshreshta, D.K., Mehta, H., Mehrotra, B.N., Patnaik, G.K. and Sharma, S.K. (1988), Indian J. Med. Plants, 87, 401-404] and inflammatory disorders [Nadkarni, K.M. (1954), Indian Materia Medica, vol.1, Popular Book Depot, Bombay pp.25-27, 619-622, 634-651, 953-955, 1220-1221, 1252-53; Dey, A.C. (1981), Indian Medicinal Plants used in Ayurvedic Preparations, Bishan Singh & Mahendra Pal Singh, Dehra Dun, India pp 81-82; Jayaweera, D.M.A. (1982) Medicinal Plants used in Ceylon, The National Science Council of Sri Lanka, Colombo, Sri Lanka, part 5, pp 76]. Hepatoprotective, Immunostimulant & Immunorestorative formulations based on P. Kurroa chemical constituents, mainly iridoid glycosides, have been developed at CDRI Lucknow & RRL Jammu [450/DEL/89 & 845/DEL/92].

Literature survey revealed that earlier reports showed the presence of trans-tetracos-15-enoic acid in Jojoba oil ex. Simmondsia chinensis seeds (0.62-1.11%) and cis isomer of the acid is reported in fatty acids of the seed oil of Microula sikkimensis (1.2%) [Wang Huiying, Yu Xuefian, Yi Yuanfen and Ding Jingkai Yunnan Zhiwu-Yanjiu 1989, 11 (1), 60-4 (Ch.), L. Jing Jingmin, Wang Jingping, Yu Fenglan, Zhiwu Xuebao, 1989, 31 (1), 50-3 (Ch.)]. These reports do not mention isolation of the constituent and the content estimation based on GLC data.

Indigofera tinctoria has been in use in indigenous system of medicine in epilepsy, nervous disorders & bronchitis [Wealth of India, vol.5. (Council of Scientific & Industrial Research, New Delhi) 182, (1959)]. The plant is also used as ointment in sores, old ulcers and haemorrhoids [R.N. Chopra, S.L. Nayar and I.C. Chopra, Glossary of Indian Medicinal Plants, 141 (1956)]. The leaves of the plants have been used in liver ailments [Nadkarni, K.M., Indian Materia Medica, vol. 1 (Popular Book Depot, Bombay, 680 (1954)]. Extract of the leaves of the plant has exhibited marked hepatoprotective effect against $CCl_4$ induced hepatic injury in rabbits, rats and mice at Regional Research Laboratory (RRL) Jammu. [Anand, K.K., Chand Dewan and Ghatak, B.J. Ray, Indian J. Exp. Biol., 17, 685 (1979); Anand, K.K., Chand Dewan, Ghatak, B.J. Ray and Arya, R.K., Indian J. Expl. Biol., 19, 298 (1981)].

Recent study in RRL Jammu for hepatoprotective effect of the plant extract and further bioactivity-guided fractionation has resulted in identification of trans-tetracos-15-enoic acid as the active principle. The constituent has been synthesized and observed to possess dose related hepatoprotective effect against galactosamine, paracetamol and $CCl_4$ as hepatotoxins using commercially available silymarin as reference material.

Activity of the formulation being described in the invention is not exactly equal to the sum of the activities of the two individual constituents and activity enhancement does not occur simply due to the mixing of the two compounds. This has been verified by mixing the formulation with RLJ-NE-299A, a standardized mixture of iridoid glycosides from Picrorhiza kurroa possessing hepatoprotective, immunostimulant and immunorestorative effects [Indian patent no. 178866].

The mixture of Apocynin, trans-tetracos-15-enoic acid prepared in many other proportions by weight have not shown any enhancement in hepatoprotective action and in some experiments the biological activity of the mixture is much less than the individual constituents.

**OBJECTS OF THE INVENTION**

The primary object of the invention is to provide a synergistic composition of transtetracos-15-enoic acid and Apocynin.

Another object of the present invention is to provide use in the treatment for hepatotoxicity.

Yet another object of the present invention is to provide a composition having broader spectrum of hepatoprotective activity than the established herbal product in use viz., Silymarin.

Still another object of the invention is to provide a composition having potential therapeutic application in obstructive and viral hepatitis.

## SUMMARY OF THE INVENTION

The present invention relates to a synergistic hepatoprotective composition containing trans-tetracos-15-enoic acid (TCA) and Apocynin (APO). The present invention also relates to use of treatment for hepatotoxicity in mammals.

## DETAILED DESCRIPTION OF THE INVENTION

Accordingly, the present invention provides a synergistic pharmaceutical composition having enhanced hepatoprotective activity on subjects, obtained from the plant *Indigofera tinctoria,* said composition comprising an effective amount of:

> (a) trans-tetracos-15-enoic acid (TCA) obtained from the plant *Indigofera tinctoria;*
> (b) Apocynin (APO) obtained from the plants Apocyanum cannabium and A. and rosaemifolium; and
> (c) the ratio of APO and TCA is in the range of 3:1 to 1:3.

An embodiment of the present invention, wherein the said composition is used either singly or in combination with pharmaceutically acceptable additives.

An another embodiment of the present invention, wherein the pharmaceutically acceptable additives are selected from the group consisting of carriers, diluents, solvents, filters lubricants, excipients, binder and stabilizers.

Yet another embodiment of the present invention, wherein the said composition is used for both preventive and curative properties.

Still another embodiment of the present invention, wherein the said composition is used systemically, orally or by any clinically/medically accepted uses.

Yet another embodiment of the present invention, wherein the said composition is used to treat hepatic disorders that are clinically, biochemically and histologically similar to that of viral hepatitis, chronic hepatitis, fatty liver, cirrhosis and several vascular lesions of the liver.

Still another embodiment of the present invention, wherein the said composition is used to treat the liver damage induced by hepatotoxins.

Yet another embodiment of the present invention, wherein the hepatotoxins are selected from the group consisting of Galactosamine, Paracetamol and Carbon tetrachloride.

Still another embodiment of the present invention, wherein the subject is selected from the group consisting of mammals.

Yet another embodiment of the present invention, wherein the dosage of said composition for the treatment of $CCl_4$ induced hepatotoxicity in mammals is 50 mg/kg-body weight.

Still another embodiment of the present invention, wherein said composition having the enhanced hepatoprotective activity in $CCl_4$ induced hepatotoxic mammals upto 92%.

Yet another embodiment of the present invention, wherein the dosage of said composition for the treatment of acetaminophen induced hepatotoxicity in mammals is 50mg/kg-body weight.

Still another embodiment of the present invention, wherein said composition having the enhanced hepatoprotective activity in acetaminophen induced hepatotoxicity in mammals upto 86%.

Yet another embodiment of the present invention, wherein said composition having the dosage of said composition for the treatment of Galactosamine induced hepatotoxicity in mammals 50 mg/kg of body weight.

Still another embodiment of the present invention, wherein said composition having the enhanced hepatoprotective activity in Galactosamine induced hepatotoxicity in mammals upto 75%.

Yet another embodiment of the present invention, the dosage of said composition for choleretic activity in mammals to control bile flow and bile solids is 50 mg/kg of body weight.

Still another embodiment of the present invention, wherein the enhanced choleretic activity is upto 39%.

The present invention also provides use in treating subjects with liver disorders with an effective amount of synergistic pharmaceutical composition to induce enhanced hepatoprotective activity, said composition comprising:

> (a) trans-tetracos-15-enoic acid (TCA) obtained from the plant *Indigofera tinctoria;*
> (b) Apocynin (APO) obtained from the plants Apocyanum cannabium and A. androsaemifolium; and
> (c) the ratio of APO and TCA is in the range of 3:1 to 1:3.

Still another embodiment of the present invention, wherein said use is used to treat liver disorders caused by Galactosamine, Paracetamol and Carbon tetrachloride.

Yet another embodiment of the present invention, a use wherein the dosage for the treatment of $CCl_4$ induced hepatotoxicity in mammals is about 50-mg/kg-body weight.

Still another embodiment of the present invention, a use wherein the enhanced hepatoprotective activity in $CCl_4$ induced hepatotoxic mammals is upto 92%.

Yet another embodiment of the present invention, a use wherein the dosage for the treatment of acetaminophen induced hepatotoxicity in mammals is 50mg/kg-body weight.

Still another embodiment of the present invention, a use wherein the enhanced hepatoprotective activity in acetaminophen induced hepatotoxicity in mammals is upto 86%.

Yet another embodiment of the present invention, a use wherein the dosage for the treatment of Galactosamine induced hepatotoxicity in mammals is 50mg/kg of body weight.

Still another embodiment of the present invention, a use wherein the enhanced hepatoprotective activity in Galactosamine induced hepatotoxicity in mammals is upto 75%.

Yet another embodiment of the present invention, a use wherein the dosage for choleretic activity in mammals to control the bile flow and bile solids is 50 mg/kg of body weight.

Still another embodiment of the present invention, a use wherein the enhanced choleretic activity in mammals is upto 39%.

Yet another embodiment of the present invention, a use wherein the composition is used either singly or in combination with pharmaceutically acceptable carriers.

Still another embodiment of the present invention, a use wherein the composition is administered to a subject in combination with pharmaceutically acceptable additives, carriers, diluents, solvents, filters, lubricants, excipients, binder or stabilizers.

Yet another embodiment of the present invention, a use wherein the desired dosage is administered for both preventive and curative properties.

Still another embodiment of the present invention, a use wherein the composition is administered systemically, orally or by any clinically/medically accepted uses.

Yet another embodiment of the present invention, a use wherein the subject is selected from animals, mammals.

The invention is further explained in the form of preferred embodiments.

i. Animals: The pharmacological studies are conducted on Wistar albino rats (150-180g) and Swiss albino mice (25-30g) of either sex, colony - bred in the Institute's animal house. After procurement, all the animals are divided into different groups and are left for one week for acclimatization to experimentation room and are maintained on standard conditions ($23 \pm 2°C$, 60-70 % relative humidity and 12 h photo period). The animals are fed with standard rodents pellet diet and water *ad libitum.* There are six animals in each group except for general behaviour and acute toxicity studies where ten animals are used in each group.

**ii. Hepatotoxins**: It is emphasized that hepatotoxin that causes acute hepatitis should have close resemblance with the viral hepatitis, clinically, biochemically and histologically. Drugs are also causes of chronic hepatic disease as chronic hepatitis, fatty liver, cirrhosis and several vascular lesions of the liver. In many instances drug induced hepatitis proves indistinguishable from viral hepatitis. Chemically induced hepatic injury for experimental studies should be severe enough to cause cell death or to modify hepatic functions. The mechanism of acute hepatic injury depends upon the chemical compound and the species of animals used. Many chemicals produce parenchymal damage, arrest bile flow and cause jaundice (chloretic injury). Hepatoprotective activity against $CCl_4$, paracetamol and D-galactosamine induced hepatotoxicity are studied.

**Carbon tetrachloride ($CCl_4$)**: $CCl_4$ is one of the most powerful hepatotoxins (in term of severity of injury). It causes toxic necrosis, which leads to biochemical changes having clinical features similar to those of acute viral hepatitis (Vogel, 1977, Bramanti *et. al.,* 1978, Kumar *et. al.,* 1992). Liver injury is produced by administration of $CCl_4$ mixed with liquid paraffin. Animals are given single dose of $CCl_4$ (50 $\mu$l.kg$^{-1}$, p.o.) in acute single treatment and (0.5 ml.kg$^{-1}$, p.o.) in case of multitreatment with drug. It is administered orally (p.o) by gastric intubation. The control animals received the equal volume of liquid paraffin. (Table 3, 4).

**Paracetamol (APAP, acetaminophen)**: It is a therapeutic agent widely used as analgesic/antipyretic drug. When taken in large doses it causes hepatic necrosis which leads to biochemical changes having clinical features similar to those of acute viral hepatitis. The similar effect is observed in animals. The toxic effect can be potentiated if it is given several hours after the anesthetic ether inhalation (Wells *et. al.,* 1986).

Liver injury is induced by injecting paracetamol (200 mg.kg$^{-1}$) interaperitoneally in normal saline (pH 9.4) six hour after inhalation of anesthetic ether (4ml/4min/6animals) in a closed chamber. The control animals received the equal volume of vehicle. (Table 2)

**D-Galactosamine**: It is one of the toxins that induce hepatic inflammatory conditions in the rat liver that clinically resembles to viral hepatitis. The mechanism of GalN induced liver injury has been extensively examined and this model is now accepted as one of the authentic systems of liver damage (Bauer *et. al.,* 1974, Al-Tuwaijiri *et. al.,* 1981). (Table1)

Hepatic damage is produced by injecting GalN (300 mg.kg$^{-1}$) subcutaneously in normal saline. The control animals received the equal volume of vehicle.

### iii. Treatment with bio-active compound and silymarin:

Freshly prepared suspension (1%, w/v) in 0.2% gum acacia in normal saline is used for all the experiments except for toxicity studies where (10 %, w/v) suspension is used. Silymarin suspension (1%, w/v) in 0.2 % gum acacia is used as a reference standard (positive control).

### iv. Experimental models:

### Effect on serum and hepatic biochemical parameters:

### CCl$_4$ induced hepatotoxicity :

**Treatment of test material before and after hepatotoxin:** The doses of TCA and APO individually and in mixture, silymarin (50 mg/kg, p.o. each) and vehicle (normal saline) are fed to different groups of rats at 48 hours, 24 hours and 2 hours before and 6 hours after hepatotoxin (CCl$_4$ , 0.5 ml.kg$^{-1}$, p.o.) intoxication. Blood is collected from orbital sinus in all the animals 18 hours after last treatment and serum separated for different estimations. All the animals are then killed by decapitation, their livers are quickly excised, cleaned of adhering tissue, weighed and homogenised in phosphate buffer saline for the analysis of hepatic parameters (Agarwal and Mehendale, 1983, Klingensmith and Mehendale, 1982, Zimmerman, 1973, Edmondson and Peter, 1985, Mitchell, et al, 1973). (Table 3-4).

### Paracetamol induced hepatotoxicity:

### Treatment of test material before and after hepatotoxin:

The doses of TCA and APO individually and in mixture, silymarin (50 mg/kg, p.o. each) and vehicle (normal saline) are fed to different groups of mice at 72 hours, 48 hours and 24 hours, 1 hour before diethyl ether inhalation and 1hour after hepatotoxin (paracetamol, 200 mg.kg$^{-1}$, i.p.) given 6 hours after exposure to diethyl- ether. Blood is collected from orbital sinus in all the animals 18 hours after last treatment and serum separated for different estimations. A portion of the liver is processed for histopathological studies. (Table 2)

### D-Galactosamine induced hepatotoxicity:

### (a) Treatment of test material before and after hepatotoxin:

The doses of TCA and APO individually and in mixture, silymarin (50 mg/kg, p.o. each) and vehicle (normal saline) are fed to different groups of mice at 48 hours, 24 hours and 2 hours before and 6 hours after hepatotoxin (GalN, 300 mg.kg$^{-1}$, s.c.) intoxication. Blood is collected from orbital sinus in all the animals 18 h after last treatment and serum separated for different estimations. All the animals are then killed by decapitation, their livers are quickly excised, cleaned of adhering tissue, weighed and homogenised in phosphate buffer saline for the analysis of hepatic parameters. A portion of the liver is processed for histopathological studies (Table 1)

### Parameters studied:

> *GPT and GOT:* Pyruvate formed by transamination reaction is determined spectrophotometrically after reaction with 2,4-dinitrophenylhydrazine (Reitman and Frankel, 1957).
> *ALP : p*-nitrophenol formed in alkaline medium is measured spectrophotometrically using p-nitrophenyl phosphate as substrate (Walter and Schutt, 1974).
> *Bilirubin* : Total bilirubin is measured by diazotization reaction with $NaNO_2$ (Malloy and Evelyn, 1937)
> *Triglycerides :* Triglycerides from serum are extracted with isopropanol and sopanified with KOH. The liberated glycerol is converted to formaldehyde by periodate and determined after reaction with acetyl acetone. Triolein is used as standard (Neri and Firings, 1973).

*Glutathione:* It is determined after deproteination by reaction with DTNB (Ellman 1959 as modified by David 1987).

*Lipid peroxidation:* Thiobarbituric acid reacting substances are determined spectrophotometrically at 535 nm. Buege. and Aust. (1978).

**Hepatoprotective activity:**

Hepatoprotective activity (H) is calculated by the following equation:

$$H = [\, 1 - (\, TC - V \,/\, VC - V \,) \,] \times 100$$

Where TC, VC, and V are drug + toxin, vehicle + toxin and vehicle treated groups of animals respectively.

**Effect on Bile flow and bile solids**

The liver, by producing bile, plays an important role in digestion. The presence of bile in the intestine is necessary to accomplish the digestion and absorption of fats as well as absorption of the fat-soluble vitamins- A, D, E & K. Bile is also an important vehicle of excretion. It removes many drugs, toxins, bile pigments and various inorganic substances either derived from the diet or synthesized by the body as cholesterol or as cholic acid. Increase in the bile flow is suggestive of stimulating action of liver microsomal enzymes.

Effect on the liver bile flow of test drug and that of vehicle is carried out after cannulating the bile duct in normal anaesthesied rats. Bile collected is from each animal from 0-5 hours (Klaassen, 1969, Donal et al.1953). (Table 5)

**Histopathological studies:**

Hitopathological studies: A portion of the liver after treatment of hepatotoxin (GalN, $CCl_4$, and paracetamol) and test material is processed for histopathological studies by routine hematoxyline and eosin stained sections (Krajian, A. A., 1963).

**General behaviour and acute toxicity:**

Using different doses (10, 30, 100, 1200, 1400, 1600, 1800 and 2000 mg.$kg^{-1}$) of said composition given orally to the groups of 10 mice for each dose, while one group with same number of mice served as control. The animals are observed continuously for 1 hour and then half hourly for 4 hours for any gross behavioral changes and general motor activity, writhing, convulsion, response to tail pinching, gnawing, piloerection, pupil size, fecal output, feeding behaviour etc. and further up to 72 hours for any mortality. Acute $LD_{50}$ values in mice are calculated by the use of Miller and Tainter, (1944). Mortality of animals in all the groups used in different models for determining hepatoprotective activity during the period of treatment is also recorded as a rough index of subacute toxicity.

**Statistical analysis**. The data obtained are subjected to statistical analysis using ANOVA for comparing different groups (Armitage, 1987) and Dunnett's *t* test for control and test groups (Dunnett, 1964). The regression coefficient (Slope b) correlation coefficient (*r*) with its p value and $ED_{50}$ with 95% confidence limit (CL) are determined by regression analysis using log dose and percent effect of adaptogenic activity (Swinscow, 1980). The two tailed paired student *t* test for comparing means before and after treatment and one tailed unpaired student *t* test for comparing control and drug treated groups (Ghosh, 1984) are used. The *p* value of < 0.05 or less is taken as the criterion of significance.

**Table 1: Hepatoprotective activity (in vivo) of TCA, APO, Mixture of TCA & APO (1:1) and silymarin (pre-treatment fed at 48 h, 24h, 2 h before and 6 h after hepatotoxin ) against the D-Galactosamine (GAIN) [(300 mg kg⁻¹ in normal saline, sub cutaneously (s.c.)] induced hepatic injury in rats[a]**

| Treatment | Dose | Serum parameters | | | | | Hepatic parameters | |
|---|---|---|---|---|---|---|---|---|
| | mg kg⁻¹ (p.o.) | GPT (Units) | GOT (Units) | ALP[b] | Bilirubin (mg %) | Triglycerides (mg %) | Lipid peroxidation[c] | Glutathione[d] |
| Vehicle Control | - | 107.18±13.48 | 112..65±3.65 | 15.63±2.02 | 0.13±0.01 | 17.16±2.07 | 28.33±2.06 | 8.46±0.77 |
| Vehicle + GalN | - | 1515.18±68.09 | 756.78±65.63 | 86.38±5.68 | 0.61±0.03 | 42.91±2.19 | 62.26±3.42 | 3.44±0.35 |
| TCA + GalN | 50 | 758.59±40.86 (53.74) | 421.64±30.36 (52.03) | 44.16±2.87 (59.67) | 0.29±0.03 (66.66) | 29.78±2.82 (50.99) | 42.12±3.93 (59.35) | 5.39±0.43 (38.84) |
| APO + GalN | 50 | 859.18±50.51 (46.59) | 509.33±41.63 (38.42) | 37.73±1.64 (68.76) | 0.41±0.02 (41.66) | 31.85±3.74 (42.95) | 48.71±3.69 (39.93) | 4.52±0.49 (21.51) |
| Mixture + GalN | 50 | 457.76±19.48 (75.10) | 309.40±26.52 (69.45) | 42.47±2.14 (62.06) | 0.31±0.01 (62.50) | 26.10±3.28 (65.28) | 39.05±3.37 (68.40) | 6.42±0.40 (59.36) |
| Silymarin + GaIN | 50 | 706.04±55.79 (57.46) | 429.35±46.94 (50.83) | 44.58±3.34 (59.08) | 0.29±0.02 (66.60) | 34.04±3.41 (34.45) | 42.81±2.52 (57.32) | 5.70±0.42 (45.02) |
| a: Values represent the mean ± S.E. and within parentheses hepatoprotective activity percent of six animals in each group, Rats: Wistar, (150-175 g) male. Unit: each unit is μmole pyruvate/min/L. b: is μ mole of *p*-nitrophenol formed/min/ L, c: is n moles MDA/g liver., d: is μ mole GSH/g liver | | | | | | | | |

**Table 2:Hepatoprotective activity (*in vivo*) of TCA, APO, Mixture of TCA & APO (1:1) and silymarin (Pre-treatment) fed at 72 h, 48 h, 24 h, 1h before inhalation of diethyl-ether and I h after 'Acetaminophen' ((APAP) 200 mg kg$^{-1}$) given i. p. 6 h after exposure to diethyl-ether in mice[a]**

| Treatment | Dose | Serum parameters | | | | | Hepatic parameters | |
|---|---|---|---|---|---|---|---|---|
| | mg kg$^{-1}$ (p.o.) | GPT (Units) | GOT (Units) | ALP[b] | Bilirubin (mg %) | Triglycerides (mg %) | Lipid Peroxidation[c] | Glutathione[d] |
| Vehicle Control | - | 137.15±14.91 | 99.69±11.02 | 18.46±1.48 | 0.12±0.01 | 15.47±0.78 | 33.64±2.53 | 6.85±0.45 |
| Vehicle+APAP | - | 2210.46±152.8 | 1124.89±90.76 | 53.52±2.98 | 0.66±0.02 | 39.21±2.22 | 57.45±2.27 | 2.93±0.33 |
| TCA+ APAP | 50 | 1008.04±63.66 (57.99) | 561.83±51.52 (54.92) | 34.95±1.89 (52.96) | 0.355±0.01 (56.48) | 23.53±1.77 (66.05) | 44..24±2.22 (55.48) | 4.59±0.45 (42.35) |
| APO + APAP | 50 | 1334.72±98.34 (42.27) | 755.04±112.04 (36.07) | 29.71±1.89 (67.91) | 0.43±0.01 (42.59) | 30.50±1.36 (36.68) | 48.32±2.13 (38.34) | 4.60±0.42 (42.60) |
| Mixture + APAP | 50 | 747.36±86.30 (70.57) | 409.27±72.84 (69.80) | 23.25±1.71 (86.33) | 0.388±0.02 (50.37) | 23.62±2.05 (65.67) | 44.12±2.64 (55.98) | 4.31±0.27 (35.20) |
| Silymarin + APAP | 50 | 1129.74±62.49 (52.12) | 671.86±69.00 (44.19) | 37.17±1.35 (46.63) | 0.373±0.02 (53.14) | 29.26±1.79 (41.91) | 42.82±2.22 (61.44) | 5.09±0.22 (55.10) |
| a: Values represent the mean ± S.E. and within parentheses hepatoprotective activity percent of six animals in each group Mice: Swiss albino (25-30 g) male. Unit: each unit is μmole pyruvate/min/L. b: is μ mole of *p*-nitroplenol formed/min/ L, c: is n moles MDA/g liver., d: is m mole GSH/g liver | | | | | | | | |

EP 1 509 285 B1

**Table 3: Hepatoprotective activity (in vivo) of TCA, APO, Mixture of TCA & APO (1:1) and silymarin (pre- treatment fed at 48h, 24h, 2h before and 6h after hepatotoxin) against $CCl_4$ (0.5 ml kg$^{-1}$, p.o.) induced hepatic injury in rats[a]**

| Treatment | Dose | Serum parameters | | | | | Hepatic parameters | |
|---|---|---|---|---|---|---|---|---|
| | mg kg$^{-1}$ (p.o.) | GPT (Units) | GOT (Units) | ALP[b] | Bilirubin (mg %) | Triglycerides (mg %) | Lipid peroxidation[c] | Glutathione[d] |
| Vehicle control | - | 118.18±29.88 | 156.97±27.97 | 23.44±2.05 | 0.15±0.02 | 15.47±0.78 | 37.75±2.7.4 | 6.96±0.59 |
| Vehicle +$CCl_4$ | - | 931.00±78.14 | 825.03±68.95 | 52.42±3.46 | 0.60±0.03 | 39.21±2.22 | 63.65±3.80 | 3.54±0.27 |
| TCA+$CCl_4$ | 50 | 324.84±42.09 (74.63) | 409.73±46.57 (62.16) | 28.72±3.90 (81.78) | 0.35±0.02 (55.55) | 23.53±1.77 (66.05) | 46.88±2.58 (64.33) | 5.35±0.51 (52.92) |
| Mixture + $CCl_4$ | 50 | 448.74±22.03 (59.33) | 472.09±38.32 (52.83) | 25.75±2.33 (92.03) | 0.30±0.02 (66.66) | 23.62±2.05 (65.67) | 49.58±3.30 (53.79) | 50.50±0.56 (44.15) |
| Silymarin + $CCl_4$ | 50 | 445.52±43.48 (59.73) | 464.95±32.39 (53.89) | 32.75±2.54 (67.87) | 0.37±0.02 (51.11) | 29.26±1.79 (41.91) | 43.49±2.07 (77.58) | 5.66±0.29 (61.98) |
| a: Values represent the mean ± S.E. and within parentheses hepatoprotective activity percent of six animals in each group, Rats: Wistar, (150-175 g) male. Unit: each unit is μmole pyruvate/min/L., b: is μ mole of *p*-nitrophenol formed/min/ L, c: is n moles MDA/g liver., d: is m mole GSH/g liver | | | | | | | | |

EP 1 509 285 B1

**Table 4: Hepatoprotective activity (in vivo) of APO (pre- treatment fed at 48h, 24h, 2h before and 6h after hepatotoxin) against $CCl_4$ (0.5 ml $kg^{-1}$, p.o.) induced hepatic injury in rats[a]**

| Treatment | Dose | Serum parameters | | | | | Hepatic parameters | |
|---|---|---|---|---|---|---|---|---|
| | mg $kg^{-1}$ (p.o.) | GPT (Units) | GOT (Units) | ALP[b] | Bilirubin (mg %) | Triglycerides (mg %) | Lipid peroxidation[c] | Glutathione[d] |
| Vehicle control | - | 87.88±11.60 | 62.86±13.16 | 21.92±1.87 | 0.13±0.02 | 10.69±0.95 | 34.62±2.69 | 6.55±0.44 |
| Vehicle+$CCl_4$ | - | 1527.57±76.47 | 765.64±75.43 | 55.90±2.43 | 0.60±0.03 | 38.03±1.65 | 58.21±3.33 | 2.99±0.41 |
| APO + $CCl_4$ | 50 | 838.58±69.65 (47.86) | 489.39±66.82 (39.31) | 26.66±2.06 (86.05) | 0.37±0.01 (48.94) | 28.00±2.39 (36.68) | 48.12±2.28 (42.77) | 4.44±0.26 (40.73) |

a: Values represent the mean ± S. E. and within parentheses hepatoprotective activity percent of six animals in each group, Rats: Wistar, (150-175 g) male.
Unit: each unit is μmole pyruvate/min/L.,
b: is μ mole ofp-nitrophenol formed/min/ L,
c: is n moles MDA/g liver.,
d: is μ mole GSH/g liver

**Table- 5: Choleretic activity of TCA, APO, Mixture ofTCA & APO (1:1) and Dehydrocholic acid (DHC) (comparative) as percent increase in bile flow and bile solids when compared to normal values in rats[a].**

| Treatment | Dose mg kg[-1] | Route | Bile parameters % Increase (as compared to normal) | |
|---|---|---|---|---|
| | | | Bile flow (ml%) | Bile solids (mg%) |
| TCA | 50 | i.d. | 08.23±1.71 | 13.40±1.53 |
| APO | 50 | i.d. | 27.39±2.05 | 24.05±2.76 |
| Mixture | 50 | i.d. | 39.46±3.73 | 37.03±4.15 |
| DHC | 50 | i.d. | 38.40±2.76 | 28.13±3.89 |

a :Values represent mean ±SE of six animals in each group, Rats: Wistar 150-175 g) male
b : Values represent mean ± SE of eight animals in each group.

## ADVANTAGES

Most of the hepatoprotective preparations/formulations available on the market are herbal based and hence are unstandardised chemically as well as biologically. Efficacy of the herbal formulations are known to be dependent upon secondary metabolites and reliability of these can only be assured if batch to batch standardization (chemical and pharmacological) are carried out.
In the present invention

a) Chemical composition of the formulation is well described, hence reproducible biological activity is assured.
b) Activity parameters are of broader spectrum and hence effectiveness of the formulation in obstructive and viral hepatitis.
c) Pharmacological evaluation data of the formulation clearly indicates synergistic action of the constituents of the formulation.

## Claims

1. A synergistic pharmaceutical composition having enhanced hepatoprotective activity on subjects, said composition comprising an effective amount of:

    (a) trans-tetracos-15-enoic acid (TCA) obtainable from the plant *Indigofera tinctoria*;
    (b) Apocynin (APO) obtainable from the plants Apocyanum cannabium and A. androsaemifolium; and
    (c) the ratio of APO and TCA is in the range of 3:1 to 1:3.

2. A composition according to claim 1 wherein said composition is used either singly or in combination with pharmaceutically acceptable additives.

3. A composition according to claim 2 wherein the pharmaceutically acceptable additives are selected from the group consisting of carriers, diluents, solvents, filters lubricants, excipients, binder and stabilizers.

4. A composition according to claim 1 wherein the said composition is used for both preventive and curative properties.

5. A composition according to claim 1 wherein said composition is to be administered systemically, orally or by any clinically/medically accepted uses.

6. A composition according to claim 1 wherein the composition is used to treat hepatic disorders that are clinically, biochemically and histologically similar to that of viral hepatitis, chronic hepatitis, fatty liver, cirrhosis and several vascular lesions of the liver.

7. A composition according to claim 1 wherein said composition is used to treat the liver damage induced by hepatotoxins.

8. A composition according to claim 1 wherein the hepatotoxins are selected from the group consisting of Galactosamine, Paracetamol and Carbon tetrachloride.

9. A composition according to claim 1, wherein subjects are selected from the group consisting of mammals.

10. A composition according to claim 8 wherein the dosage for the treatment of $CCl_4$ induced hepatotoxicity in mammals is 50 mg/kg-body weight.

11. A composition according to claim 8 wherein the enhanced hepatoprotective activity in $CCl_4$ induced hepatotoxic mammals is up to 92%.

12. A composition according to claim 8 wherein the dosage for the treatment of acetaminophen induced hepatotoxicity in mammals is 50mg/kg-body weight.

13. A composition according to claim 8 wherein the enhanced hepatoprotective activity in acetaminophen induced hepatotoxicity in mammals is up to 86%.

14. A composition according to claim 8 wherein the dosage for the treatment of Galactosamine induced hepatotoxicity in mammals is 50 mg/kg of body weight.

15. A composition according to claim 8 wherein the enhanced hepatoprotective activity in Galactosamine induced hepatotoxicity in mammals is up to 75%.

16. A composition according to claim 1 wherein the dosage for choleretic activity in mammals to control bile flow and bile solids is 50 mg/kg of body weight.

17. A composition according to claim 1 wherein the enhanced choleretic activity is up to 39%.

18. A use of synergistic pharmaceutical composition for the manufacture of a medicament for treating subjects with liver disorders with an effective amount of synergistic pharmaceutical composition to induce enhanced hepatoprotective activity, said composition comprising:

   (a) trans-tetracos-15-enoic acid (TCA) obtainable from the plant *Indigofera tinctoria;*
   (b) Apocynin (APO) obtainable from the plants Apocyanum cannabium and A. androsaemifolium; and
   (c) the ratio of APO and TCA is in the range of 3:1 to 1:3.

19. A use according to claim 18 wherein said composition is used to treat liver disorders caused by Galactosamine, Paracetamol and Carbon tetrachloride.

20. A use according to claim 19 wherein the dosage for the treatment of $CCl_4$ induced hepatotoxicity in mammals is about 50-mg/kg-body weight.

21. A use according to claim 19 wherein the enhanced hepatoprotective activity in $CCl_4$ induced hepatotoxic mammals is up to 92%.

22. A use according to claim 19 wherein the dosage for the treatment of acetaminophen induced hepatotoxicity in mammals is 50mg/kg-body weight.

23. A use according to claim 19 wherein the enhanced hepatoprotective activity in acetaminophen induced hepatotoxicity in mammals is up to 86%.

24. A use according to claim 19 wherein the dosage for the treatment of Galactosamine induced hepatotoxicity in mammals is 50mg/kg of body weight.

25. A use according to claim 19 wherein the enhanced hepatoprotective activity in Galactosamine induced hepatotoxicity in mammals is up to 75%.

26. A use according to claim 18 wherein the dosage for choleretic activity in mammals to control the bile flow and bile solids is 50 mg/kg of body weight.

27. A use according to claim 18 wherein the enhanced choleretic activity in mammals is up to 39%.

28. A use according to claim 18 wherein the composition is used either singly or in combination with pharmaceutically acceptable carriers.

29. A use according to claim 18 wherein the composition is to be administered to a subject in combination with pharmaceutically acceptable additives, carriers, diluents, solvents, filters, lubricants, excipients, binder or stabilizers.

30. A use according to claim 18 wherein the desired dosage is to be administered for both preventive and curative properties.

31. A use according to claim 18 wherein said composition is to be administered systemically, orally or by any clinically/ medically accepted uses.

32. A use according to claim 18 wherein the subject is selected from animals and mammals.


**Patentansprüche**

1. Synergistische pharmazeutische Zusammensetzung mit verbesserter hepatoprotektiver Wirksamkeit auf Probanden, wobei die Zusammensetzung eine wirksame Menge aufweist von:

   (a) trans-Tetracos-15-ensäure (TCA), erhältlich aus der Pflanze *Indigofera tinctoria*;
   (b) Apocynin (APO), erhältlich aus den Pflanzen Apocyanum cannabium und A. androsaemifolium; und
   (c) wobei das Verhältnis von APO und TCA im Bereich von 3:1 bis 1:3 liegt.

2. Zusammensetzung gemäß Anspruch 1, worin besagte Zusammensetzung entweder einzeln oder in Kombination mit pharmazeutisch akzeptablen Additiven verwendet wird.

3. Zusammensetzung gemäß Anspruch 2, worin die pharmazeutisch akzeptablen Additive aus der Gruppe, bestehend aus Trägern, Verdünnungsmitteln, Lösungsmitteln, Filtern, Gleitmitteln, Hilfsstoffen, Bindemitteln und Stabilisatoren, ausgewählt werden.

4. Zusammensetzung gemäß Anspruch 1, worin besagte Zusammensetzung sowohl für präventive, als auch für kurative Eigenschaften verwendet wird.

5. Zusammensetzung gemäß Anspruch 1, worin besagte Zusammensetzung systemisch, oral oder durch jede andere klinisch/medizinisch akzeptierte Verwendung verabreicht werden soll.

6. Zusammensetzung gemäß Anspruch 1, worin die Zusammensetzung verwendet wird, um Funktionsstörungen der Leber, die klinisch, biochemisch und histologisch denen viraler Hepatitis, chronischer Hepatitis, Fettleber, Zirrhose und verschiedenen Gefäßverletzungen der Leber gleichen, zu behandeln.

7. Zusammensetzung gemäß Anspruch 1, worin besagte Zusammensetzung verwendet wird, um den Leberschaden, der durch Hepatotoxine verursacht wird, zu behandeln.

8. Zusammensetzung gemäß Anspruch 1, worin die Hepatotoxine aus der Gruppe bestehend aus Galactosamin, Paracetamol, und Tetrachlorkohlenstoff ausgewählt sind.

9. Zusammensetzung gemäß Anspruch 1, worin Probanden aus der Gruppe, bestehend aus Säugetieren, ausgewählt sind.

10. Zusammensetzung gemäß Anspruch 8, worin die Dosierung für die Behandlung von Tetrachlorkohlenstoff-induzierter Hepatoxizität in Säugetieren 50 mg/kg-Körpergewicht beträgt.

11. Zusammensetzung gemäß Anspruch 8, worin die verbesserte hepatoprotektive Wirksamkeit in $CCl_4$-induzierten hepatoxischen Säugetieren 92% beträgt.

12. Zusammensetzung gemäß Anspruch 8, worin die Dosierung für die Behandlung von Acetaminophen-induzierter Hepatotoxizität in Säugetieren 50 mg/kg-Körpergewicht beträgt.

13. Zusammensetzung gemäß Anspruch 8, worin die verbesserte hepatoprotektive Wirksamkeit gegenüber Acetaminophen-induzierter Hepatotoxizität in Säugetieren 86% beträgt.

14. Zusammensetzung gemäß Anspruch 8, worin die Dosierung für die Behandlung von Galactosamin-induzierter Hepatotoxizität in Säugetieren 50 mg/kg-Körpergewicht beträgt.

15. Zusammensetzung gemäß Anspruch 8, worin die verbesserte hepatoprotektive Wirksamkeit gegenüber Galactosamin-induzierter Hepatotoxizität in Säugetieren 75% beträgt.

16. Zusammensetzung gemäß Anspruch 1, worin die Dosierung für choleretische Wirksamkeit in Säugetieren zur Kontrolle von Gallenfluss und Gallensteinen 50mg/kg-Körpergewicht beträgt.

17. Zusammensetzung gemäß Anspruch 1, worin die verbesserte choleretische Wirksamkeit 39% beträgt.

18. Verwendung einer synergistischen pharmazeutischen Zusammensetzung, zur Herstellung eines Medikaments, zur Behandlung von Probanden mit Lebererkrankungen mit einer wirksamen Menge der synergistischen pharmazeutischen Zusammensetzung um verbesserte hepatoprotective Wirksamkeit zu induzieren, besagte Zusammensetzung umfassend:

(a) trans-Tetracos-15-ensäure (TCA) erhältlich aus der Pflanze *Indigofera tinctoria;*
(b) Apocynin (APO) erhältlich aus den Pflanzen Apocyanum cannabium und A. androsaemifolium; und
(c) das Verhältnis von APO und TCA liegt im Bereich von 3:1 bis 1:3

19. Verwendung gemäß Anspruch 18, worin besagte Zusammensetzung verwendet wird, um Lebererkrankungen zu behandeln, welche durch Galactosamin, Paracetamol, und Tetrachlorkohlenstoff verursacht wurden.

20. Verwendung gemäß Anspruch 19, worin die Dosierung für die Behandlung von $CCl_4$-induzierter Hepatoxizität in Säugetieren 50 mg/kg-Körpergewicht beträgt.

21. Verwendung gemäß Anspruch 19, worin die verbesserte hepatoprotektive Wirksamkeit gegenüber $CCl_4$-induzierter Hepatoxizität in Säugetieren 92% beträgt.

22. Verwendung gemäß Anspruch 19, worin die Dosierung für die Behandlung von Acetaminophen-induzierter Hepatoxizität in Säugetieren 50 mg/kg-Körpergewicht beträgt.

23. Verwendung gemäß Anspruch 19, worin die verbesserte hepatoprotektive Wirksamkeit gegenüber Acetaminophen-induzierter Hepatoxizität in Säugetieren 86% beträgt.

24. Verwendung gemäß Anspruch 19, worin die Dosierung für die Behandlung von Galactosamin-induzierter Hepatoxizität in Säugetieren 50 mg/kg-Körpergewicht beträgt.

25. Verwendung gemäß Anspruch 19, worin die verbesserte hepatoprotektive Wirksamkeit gegenüber Galactosamin-induzierter Hepatoxizität in Säugetieren 75% beträgt.

26. Verwendung gemäß Anspruch 18, worin die Dosierung für choleretische Wirksamkeit in Säugetieren zur Kontrolle von Gallenfluss und Gallensteinen 50mg/kg-Körpergewicht beträgt.

27. Verwendung gemäß Anspruch 18, worin die verbesserte choleretische Wirksamkeit in Säugetieren 39% beträgt.

28. Verwendung gemäß Anspruch 18 worin besagte Zusammensetzung entweder einzeln oder in Kombination mit pharmazeutisch akzeptablen Trägern verwendet wird.

**29.** Verwendung gemäß Anspruch 18, worin die Zusammensetzung einem Probanden in Kombination mit pharmazeutisch akzeptablen Additiven, Trägem, Verdünnungsmitteln, Lösungsmitteln, Filtern, Gleitmitteln, Hilfsstoffen, Bindemitteln und Stabilisatoren verabreicht wird.

**30.** Verwendung gemäß Anspruch 18, worin die gewünschte Dosis sowohl für präventive, als auch für kurative Eigenschaften verabreicht wird.

**31.** Verwendung gemäß Anspruch 18, worin besagte Zusammensetzung systemisch, oral oder durch jede andere klinisch/medizinisch akzeptierte Verwendung verabreicht wird.

**32.** Verwendung gemäß Anspruch 18, worin der Proband aus Tieren und Säugetieren ausgewählt ist.

## Revendications

**1.** Composition pharmaceutique synergique ayant une activité hépatoprotectrice accrue sur des sujets, ladite composition comprenant une quantité efficace :

(a) d'acide trans-tétracos-15-énoïque (TCA) pouvant être obtenu à partir de la plante *Indigofera tinctoria ;*
(b) d'apocynine (APO) pouvant être obtenue à partir des plantes Apocyanum cannabium et A. androsaemifolium ; et
(c) le rapport d'APO et de TCA est dans la gamme de 3 : 1 à 1 : 3.

**2.** Composition selon la revendication 1, dans laquelle ladite composition est utilisée soit seule, soit en combinaison avec des additifs pharmaceutiquement acceptables.

**3.** Composition selon la revendication 2, dans laquelle les additifs pharmaceutiquement acceptables sont choisis dans le groupe constitué par les véhicules, les diluants, les solvants, les filtres, les lubrifiants, les excipients, les liants et les stabilisants.

**4.** Composition selon la revendication 1, dans laquelle ladite composition est utilisée pour des propriétés tant préventives que curatives.

**5.** Composition selon la revendication 1, dans laquelle ladite composition doit être administrée par voie systémique, orale ou par toute utilisation cliniquement/médicalement acceptée.

**6.** Composition selon la revendication 1, dans laquelle ladite composition est utilisée pour traiter des troubles hépatiques qui sont cliniquement, biochimiquement et histologiquement similaires à ceux de l'hépatite virale, de l'hépatite chronique, de la stéatose hépatique, de la cirrhose et de plusieurs lésions vasculaires du foie.

**7.** Composition selon la revendication 1, dans laquelle ladite composition est utilisée pour traiter l'endommagement du foie induit par des hépatotoxines.

**8.** Composition selon la revendication 1, dans laquelle les hépatotoxines sont choisies dans le groupe constitué par la galactosamine, le paracétamol et le tétrachlorure de carbone.

**9.** Composition selon la revendication 1, dans laquelle les sujets sont choisis dans le groupe constitué par les mammifères.

**10.** Composition selon la revendication 8, dans laquelle la posologie pour le traitement de l'hépatotoxicité induite par $CCl_4$ chez des mammifères est de 50 mg/kg de poids corporel.

**11.** Composition selon la revendication 8, dans laquelle l'activité hépatoprotectrice accrue chez les mammifères atteints d'une hépatotoxicité induite par $CCl_4$ va jusqu'à 92 %.

**12.** Composition selon la revendication 8, dans laquelle la posologie pour le traitement de l'hépatotoxicité induite par acétaminophène chez les mammifères est de 50 mg/kg de poids corporel.

13. Composition selon la revendication 8, dans laquelle l'activité hépatoprotectrice accrue en hépatotoxicité induite par acétominophène chez les mammifères va jusqu'à 86 %.

14. Composition selon la revendication 8, dans laquelle la posologie pour le traitement de l'hépatotoxicité induite par galactosamine chez les mammifères est de 50 mg/kg de poids corporel.

15. Composition selon la revendication 8, dans laquelle l'activité hépatoprotectrice accrue en hépatotoxicité induite par galactosamine chez les mammifères va jusqu'à 75 %.

16. Composition selon la revendication 1, dans laquelle la posologie pour l'activité cholérétique chez les mammifères pour réguler l'écoulement de bile et les solides de la bile est de 50 mg/kg de poids corporel.

17. Composition selon la revendication 1, dans laquelle l'activité cholérétique accrue va jusqu'à 39 %.

18. Utilisation d'une composition pharmaceutique synergique pour la fabrication d'un médicament destiné à traiter des sujets ayant des troubles du foie à l'aide d'une quantité efficace de composition pharmaceutique synergique pour induire une activité hépatoprotectrice accrue, ladite composition comprenant :

   (a) de l'acide trans-tétracos-15-énoïque (TCA) pouvant être obtenu à partir de la plante *Indigofera tinctoria*;
   (b) de l'apocynine (APO) pouvant être obtenue à partir des plantes Apocyanum cannabium et A. androsaemifolium; et
   (c) le rapport d'APO et de TCA est dans la gamme de 3 : 1 à 1 : 3.

19. Utilisation selon la revendication 18, dans laquelle ladite composition est utilisée pour traiter les troubles du foie provoqués par la galactosamine, le paracétamol et le tétrachlorure de carbone.

20. Utilisation selon la revendication 19, dans laquelle la posologie pour le traitement d'une hépatotoxicité induite par $CCl_4$ chez les mammifères est d'environ 50 mg/kg de poids corporel.

21. Utilisation selon la revendication 19, dans laquelle l'activité hépatoprotectrice accrue chez les mammifères atteints d'une hématotoxicité induite par $CCl_4$ va jusqu'à 92 %.

22. Utilisation selon la revendication 19, dans laquelle la posologie pour le traitement de l'hépatotoxicité induite par acétaminophène chez les mammifères est de 50 mg/kg de poids corporel.

23. Utilisation selon la revendication 19, dans laquelle l'activité hépatoprotectrice accrue en hépatotoxicité induite par acétaminophène chez les mammifères va jusqu'à 86 %.

24. Utilisation selon la revendication 19, dans laquelle la posologie pour le traitement de l'hépatotoxicité induite par galactosamine chez les mammifères est de 50 mg/kg de poids corporel.

25. Utilisation selon la revendication 19, dans laquelle l'activité hépatoprotectrice accrue en hépatotoxicité induite par galactosamine chez les mammifères va jusqu'à 75 %.

26. Utilisation selon la revendication 18, dans laquelle la posologie pour l'activité cholérétique chez les mammifères pour réguler l'écoulement de bile et les solides de la bile est de 50 mg/kg de poids corporel.

27. Utilisation selon la revendication 18, dans laquelle l'activité cholérétique accrue chez les mammifères va jusqu'à 39 %.

28. Utilisation selon la revendication 18, dans laquelle la composition est utilisée soit seule, soit en combinaison avec des véhicules pharmaceutiquement acceptables.

29. Utilisation selon la revendication 18, dans laquelle la composition doit être administrée à un sujet en combinaison avec des additifs, des véhicules, des diluants, des solvants, des filtres, des lubrifiants, des excipients, des liants ou des stabilisants pharmaceutiquement acceptables.

**30.** Utilisation selon la revendication 18, dans laquelle la posologie souhaitée doit être administrée pour des propriétés tant préventives que curatives.

**31.** Utilisation selon la revendication 18, dans laquelle ladite composition doit être administrée par voies systémique, orale, ou par toute utilisation cliniquement/médicalement acceptée.

**32.** Utilisation selon la revendication 18, dans laquelle le sujet est choisi parmi les animaux et les mammifères.